**Europäisches Patentamt**

**European Patent Office**

**Office européen des brevets**

(19)

(11) Veröffentlichungsnummer : **0 058 959**
**B1**

(12)

# EUROPÄISCHE PATENTSCHRIFT

(45) Veröffentlichungstag der Patentschrift :
21.12.88

(51) Int. Cl.⁴ : **G 01 N 33/96**

(21) Anmeldenummer : 82101284.6

(22) Anmeldetag : 19.02.82

(54) **Verfahren zur Herstellung von trübgasarmen Eich- oder Kontrollseren.**

(30) Priorität : 25.02.81 DE 3107060

(43) Veröffentlichungstag der Anmeldung :
01.09.82 Patentblatt 82/35

(45) Bekanntmachung des Hinweises auf die Patenterteilung : 21.12.88 Patentblatt 88/51

(84) Benannte Vertragsstaaten :
AT BE CH DE FR GB IT LI LU NL SE

(56) Entgegenhaltungen :
WO--A--79 /001 06
DE--A-- 2 617 742
DE--A-- 2 849 342
US--A-- 4 105 650
RESEARCH DISCLOSURE, Nr. 162, Oktober 1977, Seite 12, Nr. 16229, Havant, Hampshire, G.B.
CHEMICAL ABSTRACTS, Band 83, Nr. 18, 3. November 1975, Seite 330, Spalte 2, Nr. 152295b, Columbus, Ohio, uSA

(73) Patentinhaber : **BOEHRINGER MANNHEIM GMBH**
**Patentabteilung, Abt. E Sandhofer Strasse 112-132**
**Postfach 31 01 20**
**D-6800 Mannheim 31 Waldhof (DE)**

(72) Erfinder : Rehner, Helmut, Dr.
Am Betberg 39
D-8120 Weilheim (DE)
Erfinder : Roeschlau, Peter, Dr.
Schönegertstrasse 4
D-8124 Seeshaupt (DE)

(74) Vertreter : Weickmann, Heinrich, Dipl.-Ing. et al
Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel
Postfach 860820
D-8000 München 86 (DE)

EP 0 058 959 B1

**Beschreibung**

Die Erfindung betrifft ein Verfahren zur Herstellung von trübungsarmen Eich- oder Kontrollseren durch Mischung der gewünschten Bestandteile in wäßriger Lösung, Lyophilisierung der Lösung und Rekonstitution des Lyophilisats mit wäßrigem Medium.

Als Kontroll- oder Eichseren werden natürliche Seren humaner oder tierischer Herkunft und serumähnliche künstliche Zusammensetzungen mit genau eingestelltem Gehalt an verschiedenen interessierenden Serumbestandteilen bezeichnet, die zum Eichen oder zum Kontrolle von Verfahren zur Bestimmung von Serumbestandteilen bestimmt sind.

Der Einsatz von Human- und tierischen Seren bzw. von Zusammensetzungen ähnlicher Art, die von Seren oder Plasmen abgeleitet sind, zur Herstellung von Kontroll- und Eichseren ist bekannt. Die Einstellung der Konzentration bzw. Aktivität klinisch relevanter Parameter (Substrate, Elektrolyte, Enzyme, Hormone) auf gewünschte Werte ist ebenfalls bekannt (Proc. Royal Soc. Med. 68 (1975) 624-629).

Kontroll- bzw. Eichseren, die empfindliche Substanzen, wie Enzyme, Bilirubin, Lipoproteine, Hormone etc. enthalten, müssen aus Haltbarkeitsgründen tiefgefroren oder nach Lyophylisation bei Kühlschranktemperatur gelagert werden.

Durch den Prozeß der Lyophilisation wird die Löslichkeit einiger Komponenten verringert und infolgedessen in der rekonstituierten Probe eine Trübung erzeugt, die vor allem dann auftritt, wenn das Kontroll- oder Eichserum höhere Lipidkonzentrationen aufweist.

Dem Fachmann ist bekannt, daß Trübungen in Kontroll- bzw. Eichseren sehr unerwünscht sind. So können Probleme bei vielen spektrophotometrischen Methoden auftreten, wenn die Trübung der Probe nicht durch einen entsprechenden Probenleerwert berücksichtigt wird.

Besondere Probleme verursacht die Trübung der Kontroll- oder Eichseren vor allem bei Bestimmungen von Enzymaktivitäten, die auf der NADH/NAD$^+$-Messung beruhen und z. B. bei 340 nm durchgeführt werden.

Bei Messungen bei 340 nm kann die Trübung der Probe (Kontroll- oder Eichserum) die ohnehin schon hohe Extinktion von NADH in der Reaktionsmischung zusätzlich so stark erhöhen, daß die resultierenden Extinktionsmessungen in einem nicht mehr präzisen Extinktionsbereich des Photometers durchgeführt werden müssen. Das bedeutet, daß die Messungen ungenauer werden (d. h. der Variationskoeffizient (VK) der Bestimmungen wird größer). Weiterhin hängen die erhaltenen Ergebnisse stark von der Qualität des verwendeten Photometers ab.

Man hat bereits versucht, das Trübungsproblem auf mehrere Arten zu lösen.

1) Durch Schock-Freezing der Kontroll- bzw. Eichseren in Granulatform in einem geeigneten Gefrierbad, Lyophilisation der gefrorenen Granulate in Bulkform und Trockenabfüllung des erhaltenen Lyogranulats durch Wägung in geeigneten Flaschen (DE-OS-2 243 014).

Dieses Verfahren hat den Vorteil, daß ohne Manipulation des Kontroll-/Eichserum-Rostoffs ein Produkt relativ niedriger Trübung zu erhalten ist. Ein Nachteil besteht darin, daß diese Technologie große Investitionen verlangt und daß das Problem der Lyogranulatabfüllung zur Zeit noch nicht befriedigend gelöst, ist, d. h. technisch in größerem Umfang noch nicht durchführbar ist.

2) Durch Abtrennung der die Trübung hauptsächlich verursachenden β-Lipoproteine aus den Seren und gegebenenfalls nachträglicher Aufstockung von Cholesterin mit einer geeigneten Lipoproteinfraktion (α-Lipoprotein) oder von Triglycerid mit Trioctanoin in Gegenwart von Triton X 114 (Clin. Chem. 22 (1976) 456-460, 1299-1305).

Ein wesentlicher Nachteil dieses Verfahrens ist darin zu sehen, daß das Ausfällen der β-Lipoproteine mittels $Ca^{2+}$/Dextransulfat und ihre Abtrennung eine erhebliche Verteuerung des Serumrohstoffs zur Folge hat.

Ein anschließendes Aufstocken der Matrix mit einer geeigneten Lipoproteinfraktion zur Erhöhung der Cholesterin-Konzentration ist zwar möglich und bedingt eine mäßige Trübungszunahme (bei 300 mg Cholesterin/dl E $\gtrsim$ 0,7), aber eine gleichzeitige Erhöhung der Triglyceridkonzentration ist damit nicht möglich.

Kritisch ist ebenfalls der Zusatz von Detergens (Triton X 114). Teststörungen und/oder Enzyminaktivierungen sind hierdurch zu erwarten.

3) Durch Zusatz von Zuckern, Zuckeralkoholen und Aminozuckern zu den Kontroll- bzw. Eichseren, die die Denaturierung der Lipoproteine während der Lyophilisation verringern oder vermeiden (DE-OS-2 825 391).

Dieses Verfahren hat gegenüber den Verfahren 1) und 2) den Vorteil, daß die herkömmliche Technologie verwendet werden kann und eine Umarbeitung des Rohstoffs umgangen wird.

Nachteile dieses Verfahrens liegen darin, daß zum Teil große Mengen an « Stabilisator » zugesetzt werden müssen, wodurch sich die Eigenschaften des rekonstituierten Kontroll/Eichserums ändern (z. B. Viskosität) und daß es sich bei den Stabilisatoren um « physiologische » Stoffe handelt, die bestimmte

2

Tests stören können (z. B. Störung der Glucosebestimmung nach der HK/G6P-DH-Methode durch Glucose, Glucosamin, Fructose) oder die sich möglicherweise im Serum verändern können (z. B. Hydrolyse von Saccharose oder Lactose durch α-Glucosidase, Invertase oder β-Galactosidase, da diese Enzyme bei der Enzymaufstockung als sogenannte Enzymverunreinigungen in das Kontroll/Eichserum gelangen können).

Die Trübung (= Extinktion der unverdünnten, mit Wasser oder Diluent rekonstituierten Probe bei Hg 546 nm gegen Wasser gemessen) einer nach dem Verfahren 1) (Schock-freezing) hergestellten Probe beträgt etwa 0,5.

Die Trübung von nach konventionellem Verfahren aus normalen Humanserum hergestellten Kontroll-/Eichseren (Lyophilisation der bei — 30 °C bis — 50 °C eingefrorenen Probe) liegt bei 0,8 bis 1,3.

Kontroll-/Eichseren, die nach dem Verfahren 2) hergestellt sind, haben eine Trübung von etwa 0,3. Der Gehalt an Triglycerid bzw. Cholesterin ist jedoch nach Abtrennung der β-Lipoproteine sehr niedrig.

Aus der WO-A7900106 ist ein Verfahren bekannt, bei welchem zur Herstellung eines Kontrollserums dieses erst lyophilisiert und danach in einem Medium rekonstituiert wird, welches aus 20 bis 50 Gew.-% eines Alkylenpolyols mit 2 bis 5 Kohlenstoffatomen, Rest Wasser, besteht. Hierdurch soll die Lagerungsfähigkeit der rekonstituierten Zusammensetzung verbessert werden. Durch dieses Verfahren läßt sich die durch die Lyophilisation bedingte Herabsetzung der Löslichkeit einiger Serumskomponenten, die in der rekonstituierten Probe zur Trübungsbildung führt, nicht vermeiden.

Der Erfindung liegt daher die Aufgabe zugrunde, ein Eichserum bzw. Kontrollserum zu schaffen, welches eine verringerte Neigung zur Trübungsbildung zeigt, jedoch die Nachteile der bekannten Lösungen für dieses Problem nicht besitzt.

Es wurde nun gefunden, und hierauf beruht die Erfindung, daß sich trübungsärmere und homogenere Kontroll-/Eichseren mit der herkömmlichen Technologie herstellen lassen, wenn man zu den Ansäzten auf Human- oder Tierserumbasis oder einer sonstigen von Serum oder Plasma abgeleiteten Zusammensetzung vor der Lyophilisation bestimmte organische, nicht zu den Zuckern gehörende Verbindungen zugibt.

Erfindungsgemäß ist ein Verfahren zur Herstellung von trübungsarmen Eich- oder Kontrollseren durch Mischung der gewünschten Bestandteile in wäßriger Lösung, Lyophilisierung der Lösung und Rekonstitution des Lyophilisats mit wäßrigem Medium, dadurch gekennzeichnet, daß man vor der Lyophilisierung wenigstens eine der folgenden Verbindungen : Methanol, Natriumacetat, Tetramethyldiäthylen-Ammoniumdiacetat, Natriumpropionat, Natriumlactat, Alanin, Natrium-2,2-bis (hydroxymethyl)-propionat, N-Methyldiäthanolammonium-2,2-bis (hydroxymethyl)-propionat, 2,2-(bis-hydroxymethyl)-propionsäureamid, Natrium-2-hydroxy-2-methylbutyrat, Valin, 2-Methoxyäthanol, Triäthylenglycol, Tetraäthylenglycol, Triäthanolammoniumacetat, Tetramethyläthylendiammoniumdiacetat, Tetramethyläthylendiammonium-di-(2-hydroxyisobutyrat) und Tetramethylammoniumacetat in einer Menge von 0,5 bis 10 Gew.-%, bezogen auf die rekonstituierte Lösung, zusetzt.

Die Menge, die eine gewünschte Trübungsverbesserung ergibt, hängt weitgehend davon ab, welche Verbindung verwendet wird. Erfindungsgemäß wird eine wirksame Konzentration für Normalseren von 0,5 bis 10 g/dl angewendet. Die benötigte Menge an Einsatzstoff hängt nicht nur von ihm selbst ab, sondern wird auch von der Matrix und ihrer Zusammensetzung bestimmt (z. B. durch deren Lipid- und Proteingehalt) und kann deshalb von Fall zu Fall variieren.

Es ist auch möglich, mehrere Substanzen der oben aufgeführten Art zusammen zum Serum bzw. zu der aus Serum abgeleiteten Matrix zuzusetzen. Sich gegenseitig verstärkende oder abschwächende Effekte können hierbei auftreten.

Die Erfindung eignet sich besonders für universelle Kontroll- bzw. Eichseren, d. h. Produkte, die zur Qualitätskontrolle bzw. zum Eichen (Kalibrieren) von Automaten für möglichst alle klinisch relevanten Serumparameter, wie Enzyme, Substrate, Metabolite, Hormone, Elektrolyte usw. verwendet werden können. Ebenso eignet sich die Erfindung aber auch für Kontroll- bzw. Eichseren für spezielle Zwecke, bei denen z. B. auf den Zusatz von Enzymen oder Hormonen verzichtet werden kann. Beispiele hierfür sind Lipidkontrollserum oder Lipideichserum/Lipidkalibrator.

Erfindungsgemäß wird nicht nur das Serum als Ganzes homogener, sondern auch die Präzision von Konzentrations- und Aktivitätsbestimmungen der in den Kontroll-/Eichseren enthaltenen Parameter wird verbessert. Das bedeutet, daß die Sollwerte für die einzelnen Parameter sicherer werden und daß die Wiederfindung der deklarierten Sollwerte für den Benutzer der Qualitätskontrollseren erleichtert wird bzw. daß die Eichung der Geräte mit diesen Kalibratoren verbessert wird.

Die folgenden Beispiele erläutern die Erfindung weiter.

Die Verringerung der Trübung wird wie folgt gemessen : Die Extinktionen bei $\lambda = 546$ nm der unverdünnten, mit Wasser rekonstituierten Probe werden in einer 1 cm-Küvette gemessen und die Extinktionen von Proben mit Zusatz/Zusätzen mit Proben ohne Zusatz (= Kontrolle) verglichen.

Die Extinktion bei dieser Wellenlänge wird vorwiegend von der Trübung/Inhomogenität der Probe bestimmt, die Lichtabsorption ist relativ gering. Die Lichtintensität wird also vorwiegend durch Lichtstreuungseffekte geschwächt. Die Geometrie der Meßanordnung (Lichtweg, Anordnung von Lichtquelle und -empfänger, Blende, Küvettenform etc.) beeinflußt die durch Lichtstreuung bedingte Lichtintensitätsschwächung ebenfalls.

Die Extinktionen werden mit einem Linienspektralphotometer (Eppendorf 6115) gemessen.

**0 058 959**

Rekonstitution bzw. Rekonstitutionierung der Probe bedeutet hier den Zusatz von Wasser oder einer wäßrigen Lösung (Diluent) in der Menge zur lyophilisierten Probe, die vor Lyophilisation vorlag.

Beispiele 1 bis 15

Als Serummatrix wird in dieser Versuchsserie gepooltes Humanserum von gesunden Spender verwendet. Um den trübungsaufhellenden Effekt der zugesetzten Substanzen besser zu demonstrieren, wurde das gepoolte Serum mit Eigelbextrakt versetzt, um die Konzentration der Serummatrix an Triglyceriden auf Werte von 160 bis 270 mg/dl zu erhöhen. Der Cholesteringehalt der Serummatrix wurde nicht verändert.

Nach Zugabe der Enzyme, Substrate, Elektrolyte oder Hormone zu der Serummatrix wurden die in Tabelle 1 aufgeführten Substanzen in der angegebenen Konzentration der Serummatrix zugesetzt. Danach wurden die Ansätze mit und ohne Zusatz keimfrei filtriert (0,2 μ Membranfilterschicht), in Flaschen abgefüllt und lyophilisiert. Nach Rekonstitution mit Wasser wird die Trübung der Proben bestimmt.

Tabelle 1

| Bei- spiel | Zusatz | Konzen- tration (%) | Trübung $(E_{546})n \geq 11$ | | | |
|---|---|---|---|---|---|---|
| | | | Median | Range | $E_{max}$ | $E_{min}$ |
| 1 | Methanol | 0 | 0,82 | 0,35 | 1,07 | 0,72 |
| | | 1,5 | 0,60 | 0,07 | 0,63 | 0,56 |
| | | 3,0 | 0,28 | 0,03 | 0,30 | 0,27 |
| 2 | Natriumacetat | 0 | 0,87 | 0,23 | 0,91 | 0,78 |
| | | 2 | 0,25 | 0,02 | 0,25 | 0,23 |
| 3 | Triäthanolam- moniumacetat | 0 | 0,92 | 0,08 | 0,97 | 0,89 |
| | | 5,2 | 0,32 | 0,03 | 0,33 | 0,30 |
| 4 | Tetramethyl- diäthylenammo- niumdiacetat | 0 | 0,81 | 0,35 | 1,07 | 0,72 |
| | | 3 | 0,22 | 0,01 | 0,23 | 0,22 |
| 5 | Natriumpro- pionat | 0 | 0,87 | 0,23 | 0,91 | 0,78 |
| | | 2,4 | 0,41 | 0,09 | 0,44 | 0,35 |
| 6 | Natriumlactat | 0 | 0,87 | 0,23 | 0,91 | 0,78 |
| | | 3 | 0,16 | 0,00 | 0,16 | 0,16 |
| 7 | Alanin | 0 | 0,96 | 0,23 | 1,04 | 0,81 |
| | | 3 | 0,40 | 0,05 | 0,44 | 0,39 |
| 8 | Natrium-2,2- bis(hydroxy- methyl)-pro- pionat | 0 | 0,96 | 0,09 | 0,98 | 0,89 |
| | | 3,5 | 0,45 | 0,06 | 0,48 | 0,42 |
| 9 | N-Methyldi- äthanolammo- nium-2,2-bis- (hydroxymethyl)- propionat | 0 | 1,23 | 0,15 | 1,31 | 1,16 |
| | | 5,7 | 0,40 | 0,09 | 0,41 | 0,32 |
| 10 | 2,2-Bis(hy- droxymethyl)- propionsäure- amid | 0 | 0,96 | 0,09 | 0,98 | 0,89 |
| | | 3 | 0,67 | 0,07 | 0,69 | 0,63 |

Tabelle 1 (Fortsetzung)

| Bei-spiel | Zusatz | Konzen-tration (%) | Trübung ($E_{546}$) n ≥ 11 | | | |
| --- | --- | --- | --- | --- | --- | --- |
| | | | Median | Range | $E_{max}$ | $E_{min}$ |
| 11 | Natrium-2-hy-droxy-2-methyl-butyrat | 0 | 0,87 | 0,23 | 0,91 | 0,78 |
| | | 3 | 0,26 | 0,03 | 0,27 | 0,24 |
| 12 | Valin | 0 | 0,87 | 0,23 | 0,91 | 0,78 |
| | | 3 | 0,42 | 0,03 | 0,43 | 0,40 |
| 13 | 2-Methoxyäthanol | 0 | 1,01 | 0,11 | 1,06 | 0,95 |
| | | 3 | 0,53 | 0,03 | 0,53 | 0,50 |
| 14 | Triäthylen-glykol | 0 | 1,01 | 0,11 | 1,06 | 0,95 |
| | | 3 | 0,42 | 0,09 | 0,50 | 0,41 |
| 15 | Tetraäthylen-glykol | 0 | 1,01 | 0,11 | 1,06 | 0,95 |
| | | 3 | 0,36 | 0,10 | 0,45 | 0,35 |

Die Ergebnisse der Tabelle 1 zeigen, daß meist 3 % zugesetzte Substanz ausreichen, um die Trübung des verwendeten Humanserums deutlich zu senken. Besonders effektiv sind Methanol, Acetat, Lactat und 2-Hydroxyisobutyrat.

Weiterhin ist ersichtlich, daß die Homogenität der rekonstituierten Proben durch die Zusätze verbessert wird. Der Range ($E_{max}$ — $E_{min}$) der Trübungen von mindestens 11 einzelnen Probenflaschen wird deutlich kleiner, d. h. das Produkt wird als Ganzes einheitlicher oder homogener.

## Beispiele 16 bis 19

Für diese Versuchsserie wurde gepooltes Human- oder Rinderserum verwendet. Der Lipidgehalt der Human- bzw. Tierserummatrix ist auf 350 bzw. 150 mg Cholesterin/dl und 300 bzw. 150 mg Triglyceride/dl aufgestockt. Tabelle 2 faßt die Ergebnisse zusammen.

Tabelle 2

| Bei-spiel | Zusatz | Konzentration (g/dl) | Trübung ($E_{546}$) Median | |
| --- | --- | --- | --- | --- |
| | | | Humanserum | Rinderserum |
| | kein | - | 2,25 | 0,65 |
| 16 | TRA-acetat | 5,2 | 1,40 | 0,52 |
| 17 | TMED-(acetat)$_2$ | 3,0 | 0,55 | 0,25 |
| 18 | TMED-(2-hy-droxyisobu-tyrat)$_2$ | 4,0 | 0,51 | 0,22 |
| 19 | TMA-acetat | 3,8 | 0,31 | 0,18 |

TRA = Triäthanolammonium
TMED = Tetramethyläthylendiammonium
TMA = Tetramethylammonium

Man erkennt, daß durch eine Verbindung der Formel I mit Z = $COO^-$ zusammen mit einem geeigneten Kation die Trübung auch von lipämischen Seren stark verringert werden kann. Die Trübung von Beispiel 19 stimmt sogar mit der des nichtlyophilisierten Ausgangsmaterials überein.

**Patentanspruch**

Verfahren zur Herstellung von trübungsarmen Eich- oder Kontrollseren durch Mischung der gewünschten Bestandteile in wäßriger Lösung, Lyophilisierung der Lösung und Rekonstitution des Lyophilisats mit wäßrigem Medium, dadurch gekennzeichnet, daß man vor der Lyophilisierung wenigstens eine der folgenden Verbindungen : Methanol, Natriumacetat, Tetramethyldiäthylen-Ammoniumdiacetat, Natriumpropionat, Natriumlactat, Alanin, Natrium-2,2-bis (hydroxymethyl)-propionat, N-Methyldiäthanolammonium-2,2-bis (hydroxymethyl)-propionat, 2,2 (bis-hydroxymethyl)-propionsäureamid, Natrium-2-hydroxy-2-methylbutyrat, Valin, 2-Methoxyäthanol, Triäthylenglycol, Tetraäthylenglycol, Triäthanolammoniumacetat, Tetramethyläthylendiammoniumdiacetat, Tetramethyläthylendiammonium-di-(2-hydroxyisobutyrat) und Tetramethylammoniumacetat in einer Menge von 0,5 bis 10 Gew.-%, bezogen auf die rekonstituierte Lösung, zusetzt.

**Claim**

Process for the preparation of low turbidity calibration or control sera by mixing of the desired components in aqueous solution, lyophilisation of the solution and reconstitution of the lyophilisate with aqueous medium, characterised in that, before the lyophilisation, one adds thereto at least one of the following compounds : methanol, sodium acetate, tetramethyldiethyleneammonium diacetate, sodium propionate, sodium lactate, alanine, sodium 2,2-bis-(hydroxymethyl propionate), N-methyldiethanolammonium 2,2-bis-(hydroxymethyl) propionate, 2,2-(bis-hydroxymethyl)-propionic acid amide, sodium 2-hydroxy-2-methyl butyrate, valine, 2-methoxyethanol, triethylene glycol, tetraethylene glycol, triethanolammonium acetate, tetramethylethylenediammonium diacetate, tetramethylethylenediammonium di-(2-hydroxyisobutyrate) and tetramethylammonium acetate in an amount of 0.5 to 10 wt.%, referred to the reconstituted solution.

**Revendication**

Procédé de préparation de sérums-étalons ou de contrôle à faible turbidité par mélange en solution aqueuse des constituants désirés, lyophilisation de la solution et reconstitution du lyophilisat à l'aide d'un milieux aqueux, caractérisé par le fait qu'avant la lyophilisation on ajoute en une quantité de 0,5 à 10 % en poids par rapport à la solution reconstituée au moins l'un des composés suivants : méthanol, acétate de sodium, diacétate de tétraméthyldiéthylène-ammonium, propionate de sodium, lactate de sodium, alanine, propionate de 2,2-bis (hydroxyméthyl)-sodium, propionate de N-méthyldiéthanolammonium-2,2-bis (hydroxyméthyle), amide de l'acide 2,2 (bis-hydroxyméthyl)-propionique, butyrate de 2-hydroxy-2-méthyl-sodium, valine, 2-méthoxyéthanol, triéthylèneglycol, tétraéthylèneglycol, acétate de triéthanolammonium, diacétate de tétraméthyléthylènediammonium, di-(2-hydroxyisobutyrate) de tétraméthyléthylènediammonium et acétate de tétraméthylammonium.